# EUROPEAN PATENT SPECIFICATION

(11) **EP 1 136 032 B1**
(45) Date of publication and mention of the grant of the patent: **10.09.2003**
(21) Application number: 00850049.8
(22) Date of filing: 21.03.2000
(51) Int. Cl.: A61B 5/00

(54) **A device for measuring physical variables in a living body**
Vorrichtung zur Messung von physikalischen Grössen eines lebenden Körpers
Dispositif de mesure de variables physiques du corps humain

(43) Date of publication of application: 26.09.2001
(73) Proprietor: RADI MEDICAL SYSTEMS AB, 754 50 Uppsala (SE)
(72) Inventor: Smith, Leif, 75645 Uppsala (SE); Tenerz, Lars, 75646 Uppsala (SE); Hök, Bertil, 72344 Västeras (SE)
(74) Representative: Lindgren, Anders

(56) References cited:
- WO-A-91/08706
- US-A- 5 542 915
- US-A- 5 628 777

## Description

### FIELD OF THE INVENTION

The present invention relates to a guide wire assembly for intravascular investigations.

### TECHNICAL BACKGROUND

It is known to mount a sensor on a guide wire and to position the sensor via the guide wire in a body vessel in a living body to detect a physical parameter such as pressure or temperature. The sensor includes elements that are directly or indirectly sensitive to the parameter. For example, temperature could be measured by observing the resistance of a conductor having temperature sensitive resistance, or the length of an element having a known temperature related elongation.

US-A-5 628 777 relates to a system for intravascular measurement of a physiological variable, including a powering electronic unit, an electrical wire having a distral end for insertion into the living body and a proximal end for electrical connection to the electronic unit, a sensor connected to the distral end of the electrical wire, an internal body electrode connected to the sensor via a processing circuit, a second electrode for electrical connection to the electronic unit, the processing circuit being arranged to process the output signal from the sensor.

In order to monitor the state of the sensor inside the body some type of communication means is necessary. In some cases, means is also needed for providing power to the sensor and/or the communicating means.

Therefore, in order to power and communicate with the sensor a plurality of cables for transmitting measuring signals are connected to the sensor, and are routed along the guide wire to be passed out from the vessel to an external monitoring unit via a connector assembly. In addition, the guide wire is typically provided with a central metal wire (core wire) serving both as a support for the sensor and as a conductor of a ground potential.

However, the use of numerous cables requires that the core wire at least partly has to be replaced by a tubular section accommodating the cables. This section forms a weak part of the guide wire, and exhibits a risk for buckling during manipulation within the vessel as well as a non-symmetrical behaviour.

Also, a conventional guide wire assemblies exhibit a problem in that they require a very difficult manual assembling procedure. The extremely small components must be assembled under microscope, which is both tedious and labour intensive.

An improved guide wire, addressing some of this problems, is described in the European patent application EP 0 925 803, which forms the basis for the preambles of the independent claims of the present application. In EP 0 925 803 is described a guide wire having generally tubular conductors being disposed concentrically around the core wire, thereby allowing a simplified manufacturing process.

However, the manufacturing of such a guide wire is still comparatively time consuming and expensive and a complicated connector is required. Also, the need for an easily manipulated guide wire with a sensor still remains.

Therefore, since there is a continuous need for an easy-to-manufacture cheap and easy-to-use guide wire communication system, there remains a need for a simplified guide wire assembly including a physical property sensor.

### SUMMARY OF THE INVENTION

It is the object of the present invention to provide a system for intravascular investigation of a physiological variable inside a living body including an easy-to-manufacture and easy-to-manipulate guide wire. This object is achieved with a system according to claim 1, and a guide wire according to claim 7.

According to the invention, it is possible to reduce the number of electrical conduits of a guide wire provided with a sensor for measuring the physiological variable inside a living body, thereby providing an easy-to-manufacture and easy-to-use guide wire is provided.

Further scope of applicability of the present invention will become apparent from the detailed description given hereinafter. However, it should be understood that the detailed description and specific examples, while indicating preferred embodiments of the invention are given by way of illustration only. Various changes and modifications within the scope of the invention will become apparent to those skilled in the art from this detailed description.

### BRIEF DESCRIPTION OF THE DRAWINGS

The present invention will become more fully understood from the detailed description given herein, including the accompanying drawings which are given by way of illustration only, and thus are not limiting the present invention, and wherein
- Fig. 1: is a cross sectional view of the distal end of a guide wire according to the invention.
- Fig. 2: is a cross sectional view of the distal end of a guide wire according to another embodiment of the invention.
- Fig. 3: is a schematic view illustrating a system according to the present invention used on a patient.
- Fig. 4: is a schematic representation of a simple embodiment of the present invention.
- Fig. 5: is a block diagram of an embodiment of an electronic unit for use with the present invention.
- Fig. 6A to 6B: are interconnected time charts illustrating the decoding of a measure signal provided by a sensor unit arranged according to the invention.
- Fig. 7: in an illustration of a double wire embodiment of the present invention.
- Fig. 8: is a schematic view illustrating the embodiment of Fig. 7 used on a patient.
- Fig. 9: shows an embodiment of the present invention including an acoustical sensor.
- Fig. 10: is an impedance vs. frequency chart for an embodiment with an acoustic sensor.
- Fig. 11: is a block diagram showing an embodiment of a sensor and an associated circuitry.

### DETAILED DESCRIPTION OF EMBODIMENTS

First, a guide wire according to the present invention shall be described.

Generally, according to the invention a sensor requiring electric energy for its operation is connected at the distal end of an insulated electrical wire. Also, an internal body electrode is connected to the sensor via an electronic circuit connected to the sensor. The internal body electrode is adapted to be disposed in contact with tissue of a living body, such as blood, when inserted into the body, i.e. it has a portion for direct contact with the body tissue when inserted into the patient.

In use, the sensor is inserted into a living body while the proximal end of the electrical wire is connected to a power source and the sensor and the internal body electrode is disposed at a site inside the body where measurements are to be made. Thus, the internal body electrode is placed in contact with the living body, and specifically in contact with body tissue such as the blood, in order to obtain electrical connection therewith.

In order to obtain a closed electrical circuit a second electrode is connected to the power source and is brought to the vicinity of the internal body electrode. The second electrode has a portion for physical and electrical connection to patient, either directly or via a conductivity enhancing medium, as is well known in the art.

In one embodiment of the invention this is performed by placing the electrode on the skin of the patient, outside the site of measurement, in a manner similar to the application of an ECG electrode. In this embodiment, the electrical circuit is closed via the inner tissue and the skin of the patient.

In another embodiment of the invention the electrical circuit is closed by inserting the second electrode as well into the body in such a way that its distal end is placed near the site of measurement and is in contact with the body tissue of the patient. In this embodiment, the electrical circuit is closed internally via the tissue, i.e. typically the blood, of the patient.

It is possible to insert the sensor as well as the electrode or electrodes to any proper site within the body via a hollow tube, such as a suitable cannula or an introducer. The sensor, an additional electronic circuit, the internal body electrode, the electrical wire, and a second electrode if used, are mounted on a guide wire structure, typically for insertion via the femoral artery.

In a most preferred embodiment, the electrical wire is integrated with the core wire of the guide wire.

By using the core wire as the electrical wire the advantage of reducing the number of components is obtained, since such a metal core wire extending axially through the guide wire is normally present in the guide wire anyway, in order to provide proper stiffness to the guide wire. Thus, throughout this description the use of a guide wire having a core wire as the electrical wire will be explained for simplicity, although it is obvious that the method for communicating with the sensor described herein could be practised with a separate electrical wire running along the guide wire, or even separately, could be used as well.

The power source for use with the invention is an electronic unit situated outside of the patient's body. The electronic unit is capable of providing a suitable current through the electrical circuit partly involving the patient's body, as will be described below. In addition, the electronic unit is preferably provided with electronic circuits to interpret and present the output signal from the sensor circuit, as will be described below.

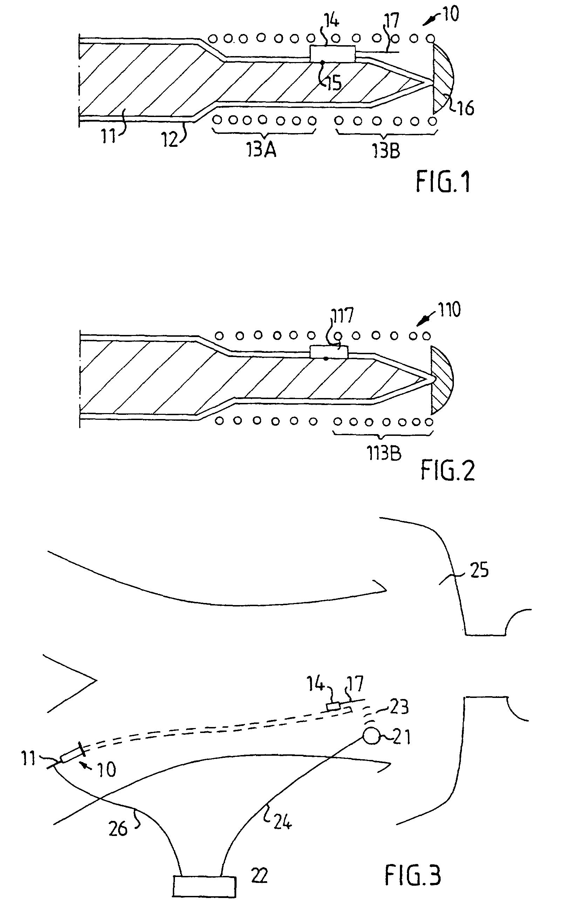

An embodiment of a guide wire of the present invention shall now be explained with reference to Fig. 1.

Fig. 1 shows a cross section of the distal end of a guide wire 10 according to the invention. A core wire 11, typically of stainless steel or a super-elastic alloy such as NITINOL® extends through the central axis of the guide wire. The core wire 11 is coated with an insulating layer 12, typically a polymer, such as Parylen®, silicone, Teflon®, polyimide, polyurethane, or a ceramic coating.

A proximal coil section 13A and a distal coil section 13B cover the distal end of the core wire. The proximal coil section 13A, which serves to provide the guide wire end with a smooth surface and a proper stiffness, is typically of stainless steel. The distal coil section 13B which is similar to the proximal section except that is also provides x-ray opacity, is typically made from platinum. Combined, the coils serve to provide a uniform outer diameter to the guide and to facilitate the introduction of the guide wire into the artery. The coils are penetrable for blood surrounding the guide wire end when inserted into an artery. The tip of the guide wire is closed by a soldered arced tip 16.

It should be noted that the coil section arrangement is described to show the shape of the distal end of a typical guide wire as used presently. However, the coil section is not necessary in itself for practising the invention although, as will be described below, it could be utilised as a part of an embodiment of the invention.

A sensor 14 is attached to the core wire, and is electrically connected thereto via a connection point 15. It should be understood that the sensor could, in addition to a component sensitive to the physical property to determine, include any electronic circuit necessary for its proper and useful function as a sensor as well as circuitry for superimposing a sensor output signal onto a carrier voltage signal.

Circuitry for such superposition purposes is in itself well known for anyone skilled in the art, and includes such devices as voltage to frequency converters, analogue to digital converters, pulse width modulators and delta modulators.

An example of an embodiment of a sensor with an associated circuitry is shown in Fig. 11, showing a rectifier 501, a sensor element 502 and a relaxation oscillator 503 connected in series. The input of the rectifier 501 is connected to the guide wire core wire via a connection point 504. The output of the relaxation oscillator 503 is connected to the internal body electrode via a connection point 505. Also, the rectifier 501 and the sensor element 502 are connected to the internal body electrode. The relaxation oscillator, as opposed to an harmonic oscillator, generates a square wave, i e the output signal flips from a minimum level to a maximum level (a digital "0" and a digital "1", respectively). The oscillating period or pulse width of such an oscillator, also known in the literature as an "astable multivibrator", can usually be controlled by a resistor-capacitor network characterised by one or several characteristic time constants RC, where R and C are resistance and capacitance values, respectively. Thus a resistive or capacitive sensor can be used to control the period time or pulse width of the oscillator.

Referring again to Fig. 1, the output signal from the sensor 14 is transferred to the surrounding blood via an output signal electrode 17. The output signal electrode 17 is at least partially uninsulated and is at one end connected to the sensor 14, to transfer the output from the sensor circuit and its other end extends into the surrounding blood between anyone of the coil sections 13A, 13B. Since the core wire is insulated the electrode 17 cannot be interfered by the potential of the core wire.

An alternative embodiment of the output signal electrode 117 is shown in Fig.2, wherein components differing from the components of Fig. 1 have referral numbers. According to Fig. 2, the output signal electrode 117 is connected to, or forms a part of, one of the coil sections 113B. While anyone of the coil sections are useful, at least a part of the selected section should not be insulated in order to allow contact with the surrounding body fluids. With this alternative embodiment a simple design that guarantees a proper contact with the surrounding parts of the body is obtained.

The use of a guide wire 10 according to the present invention, such as is illustrated in Fig. 1, is schematically shown in Fig. 3. The guide wire 10 is inserted into the femoral artery of a patient 25. The position of the guide wire 10, the sensor 14 and the output signal electrode 17 inside the body is illustrated with dotted lines.

An external electrode 21, generally similar to an ECG electrode, is attached to the skin of the patient 25 near the position for the sensor circuit 14. The external electrode 21 is coupled to an electronic unit 22 via an electrical wire 24. The guide wire 10, and more specifically the core wire 11 thereof, is also coupled to the electronic unit 22 via a wire 26 that is connected to the core wire using any suitable connector means (not shown), such as a connector of crocodile clip type or any other known connector.

The electronic unit 22 provides an electrical voltage to the circuit comprising the wire 26, the core wire 11 of the guide wire 10, the sensor circuit 14 the output signal electrode 17, through the blood and other tissue 23 of the patient, the electrode 21 and the wire 24, respectively.

In use, the sensor 14 is inserted into the patient, for example as has been described above with reference to Fig. 3, and an electrode 21 is applied approximately above the output signal electrode 17, for example via the patient's skin as has also been described. The method to introduce the guide wire, as well as the method necessary to provide a proper electrically conducting attachment of the electrode applied to the skin of the patient are well known to those skilled in the art. The core wire 11 and the electrode 21 are connected to the electronic unit 22.

The currents being generated through the patient's tissue due to the applied electric voltage have to be low enough to be safely transferred through the human body. Allowable values are preferably selected according to the international standard IEC 601-1 (1988), clause 19. Although a weak DC voltage could be used, it is preferred to use an AC voltage in order to obtain high useful currents through the body, such as an alternating voltage with a frequency higher than 1 kHz which makes it possible to allow currents up to 10 mA without risks for the health of the patient.

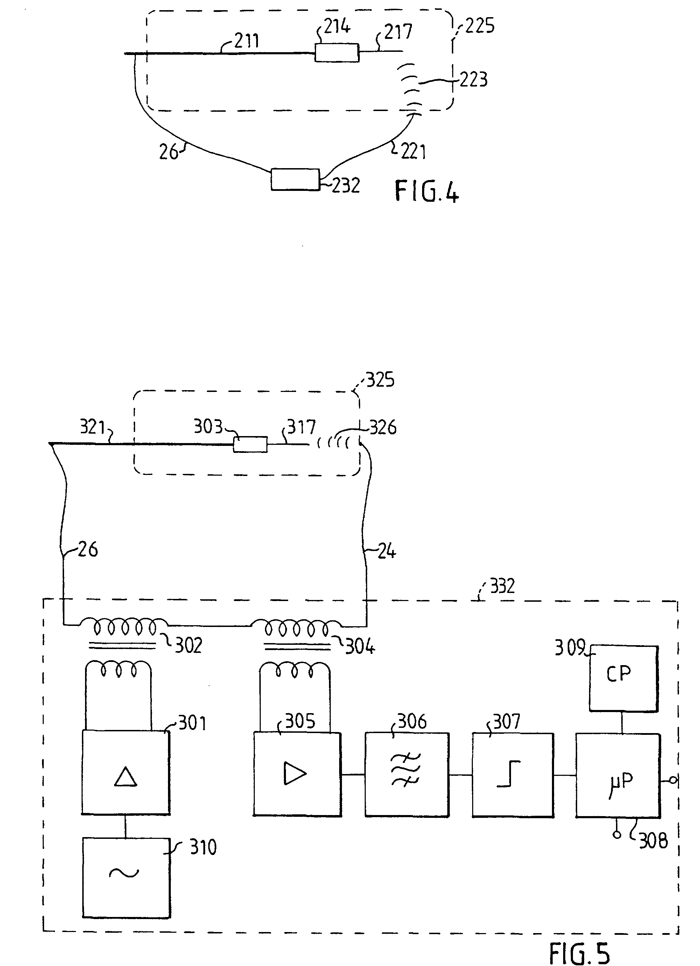

In a very simple embodiment, corresponding to the description above and being schematically shown in Fig. 4, for measuring the temperature within a body the sensor is a temperature sensitive resistor 214, having a known relationship between temperature and resistance and at one end (corresponding to the connecting point 15 of Fig. 1) connected to a core wire 211 and at the other end connected to an output signal electrode 217. The sensor is inserted into a body, which is very schematically indicated with a dotted line 223. An electronic unit 232 outside of the body provides a voltage to the sensor 214 via a conduit 26, the core wire 211 and via the output signal electrode 217, the body tissue 233 and an electrode 221 disposed on the skin of the body 223.

The voltage provided by the electronic unit 232 could be an AC or a DC voltage. The electronic unit 232 also includes means for registering the overall resistance of the circuit via the body. Such means are well known, and will not be described herein.

By measuring the overall resistance, and knowing the temperature-resistance relationship of the sensor, the temperature at the sensor could easily be calculated.

The main advantage of this embodiment is simplicity. However, it has the drawback of low accuracy and reliability. These disadvantages are a result of influences from other resistances within the monitored circuit, for example the coupling impedance of the skin electrode 221.

Generally, in the case of applying an AC voltage the sensor is typically connected to a circuit that includes a rectifier that transforms the AC voltage to a DC voltage for driving the sensor selected to be sensitive to the physical parameter to be investigated. An example of a sensor for use in such an application and useful for measuring cardiovascular pressure, is described in Transducers '87 (The 4th international conference on solid state sensors and actuators), p. 344, "An ultraminiature solid-state pressure sensor for a cardiovascular catheter" by H. Chau and K. D. Wise.

As described above, the output signal of the sensor is processed by a circuit connected to the sensor in such a way that information representing the level of the monitored physical parameter is superimposed on the signal that is provided by the electronic unit.

The electronic unit includes circuitry to calculate the value of the investigated parameter based on the superimposed signal.

An embodiment of such an electronic unit 332 is shown in Fig. 5, wherein the guide wire assembly is schematically represented by a core wire 321, a sensor unit 303 and an internal body electrode 317 for providing an electrical signal via the tissue 326 of a patient. The guide wire assembly is introduced into the patient (the patient being suggested with a dotted line 325). The electronic unit 332 is connected to the core wire 321 via a cable 26, and to the outside of the body via the tissue and a second cable 24.

The electronic unit 332 comprises a drive oscillator 310, providing an AC voltage typically in the range of 2-10 V at a frequency in the range of 100 kHz - 1MHz, connected to a drive amplifier 301 which drives a first transformer 302. The first transformer 302 is non-galvanically coupled to the circuit through the body, to provide a feeding voltage.

A second transformer 304, which is also coupled to the circuit through the body in a non-galvanic way, is used for detection of the superimposed signal. The signal is amplified with an amplifier 305, and a narrow band-pass filter 306 is used to eliminate low and high frequency interference. The band-pass filter could for instance be a so called phase-sensitive amplifier or a synchronous amplifier.

A Schmitt-trigger (or comparator) 307 is connected to the output of the band-pass filter 306, to trigger (send a digital "1") at a selected voltage threshold level. A digital micro-processor 308 correlates the trigger pulses with a clock pulse generator 309 to count the number of clock pulses between consecutive trigger pulses.

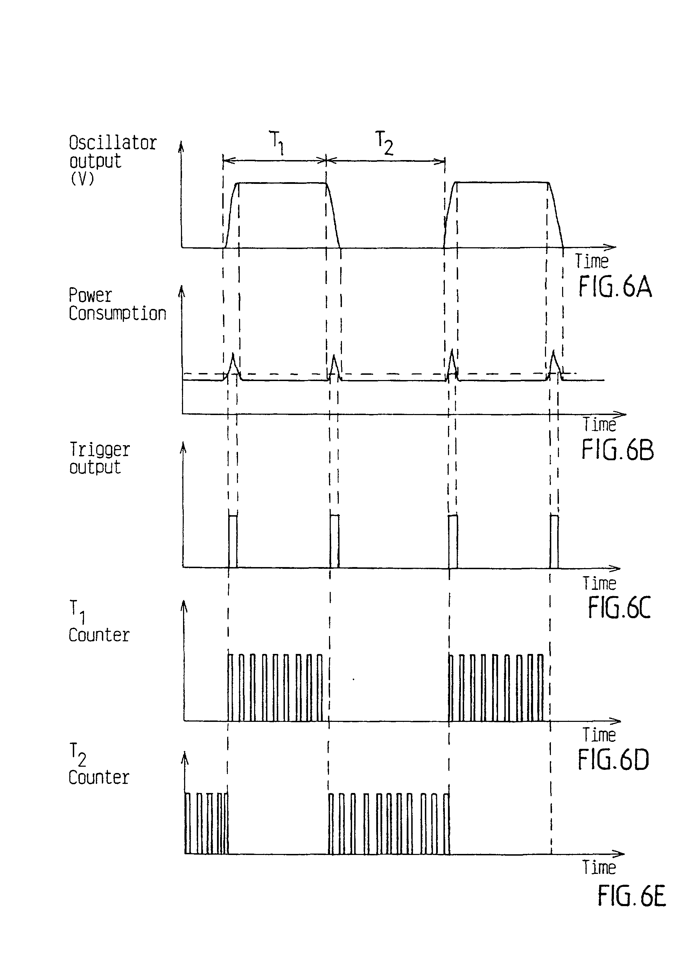

Figs. 6A to 6E, which are interconnected with respect to a time axis, illustrate the decoding of a measure signal provided by the sensor unit 303 in a set-up according to Fig. 5. The sensor unit 303 is a unit including an oscillator, corresponding to the sensor circuit described above with reference to Fig. 11.

Fig. 6A shows the oscillator output signal from the sensor unit 303. The measuring conditions, i.e. the measured variable (or variables), determines the pulse width T1 and T2.

Fig. 6B shows the power consumption of the sensor unit 303. The power consumption is essentially constant with superimposed peaks coinciding in time with the sensor oscillator's transition from "0" to "1", or vice verse.

Fig. 6C shows the output signal from the Schmitt-trigger 307. The trigger threshold is shown as a horizontal dotted line in Fig. 6B.

Fig. 6D illustrates the clock pulses from the clock pulse generator 309 being calculated by the micro processor 308 to determine the time interval T1.

Fig. 6E illustrates the clock pulses from the clock pulse generator 309 being calculated by the micro processor 308 to determine the time interval T2.

Thus, by determining the time intervals coded by the sensor and its corresponding circuit, information on the measured physiological variable can be obtained via an electrical signal passing through the body tissue.

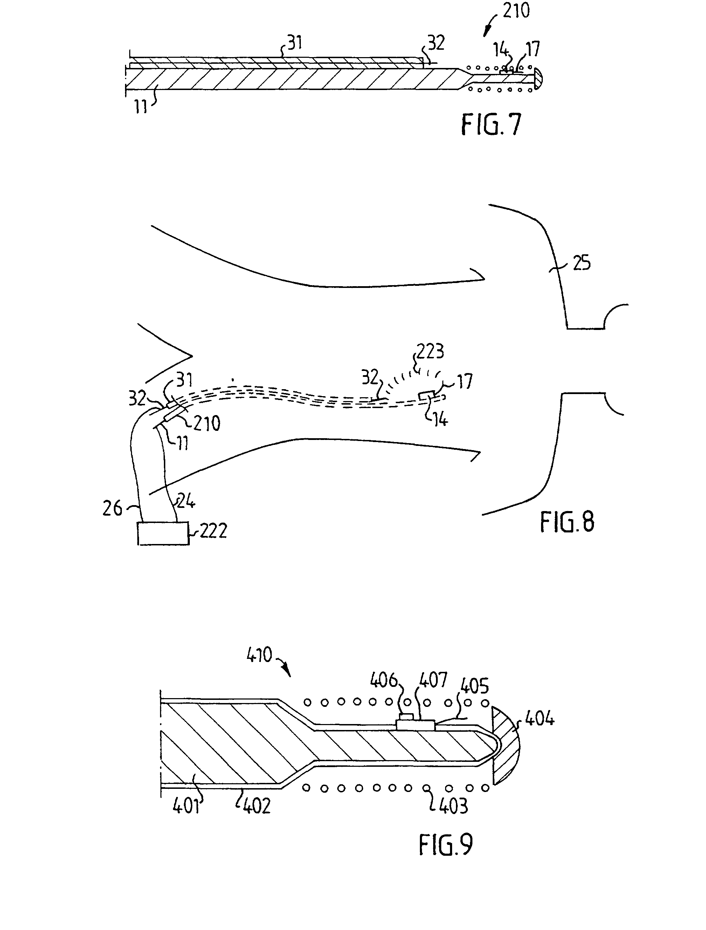

In Fig. 9 is shown an embodiment of the present invention including an acoustical sensor. As is shown with the previous embodiments, a guide wire 410 is provided with a core wire 401 covered with an insulating layer 402. The distal end of the guide wire has a rounded tip 404 and a coil 403 acting as an output signal electrode according to the invention, similar to what is previously described.

A piezo-ceramic plate 407, such as a PZT (lead zirconate-titanate) plate, is connected to the core wire and to the output signal electrode 403. A micro-mechanical acoustic resonator 406 is attached to the piezo-ceramic plate 407. The resonator should be selected such that its resonance frequency is dependent on the physiological variable to be measured.

Examples of useful micro-mechanical acoustic resonators are described in US 5,188,983, "Polysilicon resonating beam transducers and methods of producing the same" to H Guckel et al.

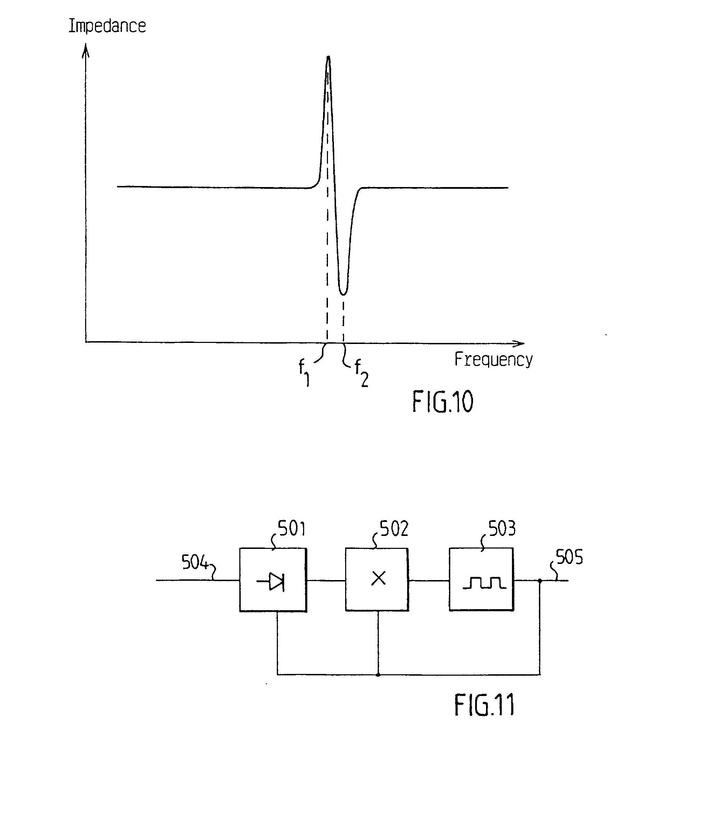

When the piezo-ceramic plate 407 is energised by alternating voltage provided via the body tissue, as described above, it responds with mechanical vibrations that are transferred to the resonator 406. At the resonance frequencies f1 or f2 (corresponding to serial or parallel resonance), as illustrated in Fig. 10, a peak and a valley point, respectively, appears in the electrical impedance. The electrical impedance is detectable by an external electronic unit, corresponding to the unit described above with reference to Fig. 5, and consequently the physiological variable sought for could be calculated.

Thus, the guide wire assembly and the communicating system according to the invention, for determining a physical parameter inside the body of a patient, enables the transfer of information regarding a physiological variable detected by a sensor inside the body using a single electrical wire provided in the guide wire. According to the invention, this is obtained by using tissue of the patient, such as the blood and the skin, to act as a conductor in co-operation with a guide wire according to the invention.

A guide wire assembly according to the present invention provides for a very simple manufacturing, using few components. The circuitry of the sensor, with the associated internal body electrode, is easily connected to an exposed section of the core wire at the distal end of the guide wire using any suitable conventional method, such as soldering. No additional cables are called for, allowing the core wire to be connected to the electronic unit with any suitable connector means.

In addition, as is shown in the embodiments described above, the present invention allows a sensor guide wire design wherein the absence of other conduits than the core wire provides a suitable flexibility and symmetry to allow good manoeuvrability during positioning of the guide wire in a vessel.

Another embodiment of the invention, herein called the double wire embodiment, is illustrated in the schematic illustration of Fig. 7. According to Fig. 7, the guide wire 210 is similar to the guide wire 10 shown in Fig. 1, for example having an insulated core wire 11, but is in addition thereto provided with a second insulated wire 31. At a distal portion, near the distal end of the guide wire, the insulation is removed from the second wire 31 to expose the conductor to form an electrode 32.

When inserted into the patient's body 25, as is shown in Fig. 8, the core wire 11 of the guide wire 210 is connected to an electronic unit 222, similar to the electronic unit 22 of the first embodiment. The second electrical wire 31 is also connected to the electronic unit 222. At the same time the electrode end 32 of the second insulated wire is inserted in the body with the guide wire to contact the body fluids of the patient. In use, the electric circuit including the core wire 11, the sensor (and any additional electronic circuits) 14, the output signal electrode 17, the conductor 31 and its electrode end 32, the connecting leads 24, 26 and the electronic unit 222 is closed via the body tissue 23, i.e. the blood, of the patient.

Thus, similarly to what has been explained for the previous embodiments, the electric circuit includes tissue of the patient's body. However, in the double wire embodiment no electricity is transferred through the skin of the patient and no electrode is applied on the skin of the patient. Instead, the electric signals leaving the output signal electrode 17 propagates through the surrounding blood to the second electrical wire 31.

Thus, with the double wire embodiment it is not necessary to apply an electrode to the skin of the patient, although this is obtained to the cost of a more complicated and hard to manipulate guide wire.

Although it is necessary to connect the guide wire according to the second embodiment using two connectors, anyone of these connectors could be any suitable connector, such as a simple low-cost connector of crocodile type.

## Claims

1. A system for intravascular measurement of a physiological variable inside a living body, including a powering electronic unit (22; 222; 332), an electrical wire (11) having a distal end for insertion into the living body and a proximal end for electrical connection to the electronic unit (22; 222; 332), and a sensor (14; 502; 406) arranged to perform said measurement and connected to the distal end of the electrical wire,
wherein
an internal body electrode (17; 117, 113B; 317;405) is connected to the sensor (14; 502; 406) via a processing circuit, said internal body electrode having a portion for physical and electrical contact with body tissue when disposed at the site of measurement; and
a second electrode (21, 24; 221; 32) for electrical connection to the electronic unit (22; 222; 332), said second electrode having a portion for electrical contact with a second part of the living body,
said processing circuit is arranged to process the output signal from the sensor and to apply the processed electrical signal to said internal body electrode, said system thus being adapted to transfer said processed electrical signal from said internal electrode, via the body tissue, to said second electrode, wherein said processed electrical signal includes information regarding said measured physiological variable.

2. The system according to claim 1, wherein the electrical wire (11) for insertion into the body is a core wire extending through a guide wire.

3. The system according to claim 1 or 2, wherein the electronic unit (22; 222; 332) comprises an AC power source to power the sensor with an AC voltage via the body tissue, wherein said AC voltage is within the range of 2-10 V, and is of a frequency in the range of 100 kHz - 1MHz.

4. The system according to claim 1, wherein said processing circuit superimposes the sensor output signal onto a carrier voltage signal.

5. The system according to claim 1 or 4, wherein said processing circuit includes a rectifier and a relaxation oscillator that are used to process the output signal such that the processed signal is provided with a variable period time or pulse width in dependence of the output signal.

6. The system according to anyone of the previous claims, wherein said second electrode is a second electrical wire (31) having an electrode portion (32) at its distal end for insertion into the living body.

7. A guide wire for intravascular measurement of a physiological variable inside a living body, including an electrically conducting wire (11) extending along the guide wire and a sensor (14; 502; 406) at the distal end of the guide wire connected to the electrically conducting wire (11) and arranged to perform said measurement,
wherein
an internal body electrode (17; 117, 113B; 317;405) is connected to the sensor (14; 502; 406), said internal body electrode having a portion for physical and electrical contact with body tissue when disposed at the site of measurement, said sensor is provided with a circuitry (501,502,503) including a rectifier and a relaxation oscillator used to process the output signal from the sensor such that the processed signal is provided with a variable period time or pulse width in dependence of the output signal, said processed signal is adapted to be applied to said internal body electrode, wherein said processed signal includes information regarding said measured physiological variable.

8. The guide wire according to claim 7, wherein the electrically conducting wire (11) is a core wire extending through the guide wire.

9. The guide wire according to claim 7 or 8, wherein a coil (113B) covering the distal end of said guide wire is connected to the sensor to act as said internal body electrode.

10. The guide wire (210) according to anyone of claims 7 or 9, **characterised in that** a second electrical wire (31) is attached thereto to extend along the guide wire, said second electrical wire having an electrode portion (32) near the distal end of the guide wire for physical and electrical contact with body tissue.

## Patentansprüche

1. System zur intravaskulären Messung einer physiologischen Variablen innerhalb eines lebenden Körpers, eine Stromversorgungselektronikeinheit (22; 222; 332) einschließend, einen elektrischen Draht (11) mit einem distalen Ende zum Einführen in den lebenden Körper und einem proximalen Ende zum elektrischen Verbinden mit der Elektronikeinheit (22; 222; 332) und einen Sensor (14; 502; 406), der eingerichtet ist zum Durchführen der Messungen und mit dem distalen Ende des elektrischen Drahtes verbunden, wobei
eine interne Körperelektrode (17; 117; 113B; 317; 405) mit dem Sensor (14; 502; 406) über eine Verarbeitungsschaltung verbunden ist, wobei die interne Körperelektrode einen Abschnitt zum physikalischen und elektrischen Kontaktieren mit Körpergewebe hat, wenn am Messort angeordnet; und
eine zweite Elektrode (21, 24; 221; 32) zum elektrischen Verbinden mit der Elektronikeinheit (22; 222; 332),
wobei die zweite Elektrode einen Abschnitt zum elektrischen Kontaktieren mit einem zweiten Teil des lebenden Körpers hat,
wobei die Verarbeitungsschaltung eingerichtet ist zum Verarbeiten des Ausgangssignals von dem Sensor und zum Anlegen des verarbeiteten elektrischen Signals an die interne Körperelektrode, wobei das System derart eingerichtet ist um das verarbeitete elektrische Signal von der internen Elektrode über das Körpergewebe zu der zweiten Elektrode zu übertragen, wobei das verarbeitete elektrische Signal Information bezüglich der gemessenen physiologischen Variablen einschließt.

2. System nach Anspruch 1, wobei der elektrische Draht (11) zum Einfügen in den Körper ein sich durch einen Führungsdraht erstreckender Kerndraht ist.

3. System nach Anspruch 1 oder 2, wobei die Elektronikeinheit (22; 222; 332) eine Wechselstromenergiequelle umfasst zum Versorgen des Sensors mit einer Wechselspannung über das Körpergewebe, wobei die Wechselspannung im Bereich von 2-10 V liegt mit einer Frequenz im Bereich von 100 kHz - 1 MHz.

4. System nach Anspruch 1, wobei die Verarbeitungsschaltung das Sensorausgangssignal einem Trägerspannungssignal überlagert.

5. System nach Anspruch 1 oder 4, wobei die Verarbeitungsschaltung einen Gleichrichter einschließt und einen Relaxasitionsoszillator, die verwendet werden zum Verarbeiten des Ausgangssignals derart, dass das verarbeitete Signal mit einer variablen Periodenzeit oder Pulsbreite in Abhängigkeit von dem Ausgangssignal versehen ist.

6. System nach einem der vorhergehenden Ansprüche, wobei die zweite Elektrode ein zweiter elektrischer Draht (31) mit einem Elektrodenabschnitt (32) an seinem fernen Ende zum Einfügen in den lebenden Körper ist.

7. Führungsdraht zur intravaskularen Messung einer physiologischen Variablen innerhalb eines lebenden Körpers, einen elektrischen Leitungsdraht (11) einschließend, der sich entlang des Führungsdrahtes erstreckt und einen Sensor (14; 502; 406) am distalen Ende des Führungsdrahtes verbunden mit dem elektrischen Leitungsdraht (11) und eingerichtet zum Durchführen der Messung,
wobei eine interne Körperelektrode (17; 117; 113B; 317; 405) mit dem Sensor (14; 502; 406) verbunden ist, wobei die interne Körperelektrode einen Abschnitt zum physikalischen und elektrischen Kontaktieren mit Körpergewebe hat, wenn an dem Messort angeordnet, wobei der Sensor mit einer Schaltung (501, 502, 503) versehen ist, einschließlich eines Gleichrichters und eines Relaxasitionsoszillators, verwendet zum Verarbeiten des Ausgangssignals von dem Sensor derart, dass das verarbeitete Signal mit einer variablen Periodendauer oder Pulsbreite in Abhängigkeit von dem Ausgangssignal bereitgestellt wird, wobei das verarbeitete Signal eingerichtet ist, um angelegt zu werden an die interne Körperelektrode, wobei das verarbeitete Signal Information bezüglich der gemessenen physiologischen Variablen einschließt.

8. Führungsdraht nach Anspruch 7, wobei der elektrisch leitfähige Draht (11) ein Kerndraht ist, der sich durch den Führungsdraht erstreckt.

9. Führungsdraht nach Anspruch 7 oder 8, wobei eine Spule (113B) zum Abdecken des distalen Endes des Führungsdrahtes mit dem Sensor verbunden ist, um als interne Körperelektrode zu agieren.

10. Führungsdraht (210) nach irgendeinem der Ansprüche 7 oder 9, **dadurch gekennzeichnet, dass** ein zweiter elektrischer Draht (31) daran angebracht ist, um sich entlang des Führungsdrahtes zu erstrecken wobei der zweite elektrische Draht einen Elektrodenabschnitt (32) in der Nähe des distalen Endes des Leitungsdrahtes hat zum physikalischen und elektrischen Kontaktieren mit Körpergewebe.

## Revendications

1. Système pour la mesure intravasculaire d'une variable physiologique à l'intérieur d'un corps humain, comprenant une unité électronique d'alimentation (22 ; 222 ; 332), un fil électrique (11) comportant une extrémité distale destinée à être insérée dans le corps humain et une extrémité proximale destinée à la connexion électrique à l'unité électronique (22 ; 222 ; 332), et un capteur (14 ; 502 ; 406) agencé pour effectuer ladite mesure et connecté à l'extrémité distale du fil électrique,
dans lequel
une électrode corporelle interne (17 ; 117, 113B ; 317 ; 405) est connectée au capteur (14 ; 502 ; 406) par l'intermédiaire d'un circuit de traitement, ladite électrode corporelle interne comportant une partie destinée à un contact physique et électrique avec un tissu cellulaire lorsqu'elle est disposée à l'endroit de mesure ; et
une deuxième électrode (21, 24 ; 221 ; 32) pour la connexion électrique à l'unité électronique (22 ; 222 ; 332), ladite deuxième électrode comportant une partie destinée à un contact électrique avec une deuxième partie du corps humain,
ledit circuit de traitement est agencé pour traiter le signal de sortie provenant du capteur et pour appliquer le signal électrique traité à ladite électrode corporelle interne, ledit système étant ainsi adapté pour transférer ledit signal électrique traité de ladite électrode interne, par l'intermédiaire du tissu cellulaire, à ladite deuxième électrode, dans lequel ledit signal électrique traité comprend des informations concernant ladite variable physiologique mesurée.

2. Système selon la revendication 1, dans lequel le fil électrique (11) destiné à être inséré dans le corps est un fil central s'étendant à travers un fil de guidage

3. Système selon la revendication 1 ou 2, dans lequel l'unité électronique (22 ; 222 ; 332) comprend une source d'alimentation alternative pour alimenter le capteur avec une tension alternative par l'intermédiaire du tissu cellulaire, dans lequel ladite tension alternative se situe dans la plage de 2 à 10 V et a une fréquence comprise dans la plage de 100 kHz à 1 MHz.

4. Système selon la revendication 1, dans lequel ledit circuit de traitement superpose le signal de sortie de capteur à un signal de tension de porteuse.

5. Système selon la revendication 1 ou 4, dans lequel ledit circuit de traitement comprend un redresseur et un oscillateur à relaxation qui sont utilisés pour traiter le signal de sortie de telle sorte que le signal traité soit pourvu d'un intervalle de temps ou d'une durée d'impulsion variable dépendant du signal de sortie.

6. Système selon l'une quelconque des revendications précédentes, dans lequel ladite deuxième électrode est un deuxième fil électrique (31) comportant une partie d'électrode (32) au niveau de son extrémité distale destinée à l'insertion dans le corps humain.

7. Fil de guidage pour la mesure intravasculaire d'une variable physiologique à l'intérieur d'un corps humain, comprenant un fil électriquement conducteur (11) s'étendant le long du fil de guidage et un capteur (14 ; 502 ; 406) au niveau de l'extrémité distale du fil de guidage connecté au fil électriquement conducteur (11) et agencé pour effectuer ladite mesure,
dans lequel
une électrode corporelle interne (17 ; 117, 113B ; 317 ; 405) est connectée au capteur (14 ; 502 ; 406), ladite électrode corporelle interne comportant une partie destinée à un contact physique et électrique avec un tissu cellulaire lorsqu'elle est disposée à l'endroit de la mesure, ledit capteur est pourvu d'éléments de circuit (501, 502, 503) comprenant un redresseur et un oscillateur à relaxation utilisés pour traiter le signal de sortie provenant du capteur de telle sorte que le signal traité soit pourvu d'un intervalle de temps ou d'une durée d'impulsion variable dépendant du signal de sortie, ledit signal traité est adapté pour être appliqué à ladite électrode corporelle interne, dans lequel ledit signal traité comprend des informations concernant ladite variable physiologique mesurée.

8. Fil de guidage selon la revendication 7, dans lequel le fil électriquement conducteur (11) est un fil central s'étendant à travers le fil de guidage.

9. Fil de guidage selon la revendication 7 ou 8, dans lequel une bobine (113B) recouvrant l'extrémité distale dudit fil de guidage est connectée au capteur pour agir en tant que dite électrode corporelle interne.

10. Fil de guidage (210) selon l'une quelconque des revendications 7 ou 9, **caractérisé en ce qu'**un deuxième fil électrique (31) est fixé à celui-ci de manière à s'étendre le long du fil de guidage, ledit deuxième fil électrique comportant une partie d'électrode (32) à proximité de l'extrémité distale du fil de guidage destinée à un contact physique et électrique avec un tissu cellulaire.
